# EUROPEAN PATENT APPLICATION

(11) **EP 4 431 026 A1**
(43) Date of publication of application: **18.09.2024**
(21) Application number: 24162636.5
(22) Date of filing: 11.03.2024
(51) Int. Cl.: A61B 10/00

(54) **DEVICE WITH SECONDARY DETERMINATION CHAMBER FOR TESTING ANALYTE IN LIQUID SAMPLE**

(30) Priority: 13.03.2023 CN 202310257428; 22.03.2023 US 202363453899 P
(71) Applicant: Zhejiang Orient Gene Biotech Co., Ltd, Anji Huzhou Zhejiang 313300 (CN)
(72) Inventor: LEI, Siyu, Huzhou, 313300 (CN); SHEN, Lili, Huzhou, 313300 (CN); FANG, Jianqiu, Huzhou, 313300 (CN)
(74) Representative: Piotrowicz, Pawel Jan Andrzej

(57) **Abstract**

The present invention provides a device for detecting an analyte in a liquid sample, and the device includes a first chamber for collecting the liquid sample and a second chamber for collecting a liquid sample for determination test; where the first chamber is detachably combined or connected with the second chamber; the first chamber is in communication with the second chamber; and the liquid sample located in the second chamber is a part of the liquid sample in the first chamber; in some embodiments, the second chamber is connected with one traction mechanism, which helps the second chamber depart from the first chamber.

## Description

### CROSS-REFERENCE TO RELATED APPLICATIONS

This application claims priority to the Chinese Application, Application No. 202310257428.3, filed on March 13, 2023, and to the U.S Provisional Application, Application No. 63/453, 899, filed on March 22, 2023, and all contents of the Applications are hereby incorporated by reference in their entirety as a part of the present invention.

### BACKGROUND OF THE INVENTION

### Field of the Invention

The present invention relates to a device for collecting a liquid sample, and in particular, to a device for collecting and testing an analyte in a liquid sample in the field of rapid diagnosis, such as a urine collection and testing device, and especially to a device with a secondary determination chamber for secondary determination test of samples.

### Description of the Related Art

At present, the device for testing the presence or absence of an analyte in sample is widely used in hospitals or homes, and such testing devices for rapid diagnosis include one or more test strips, for example, for early pregnancy testing and drug of abuse testing. Such testing devices for rapid diagnosis are very convenient, and can obtain testing results from the test strips in one minute or at most ten minutes or so.

Drug tests are widely used by the drug control department, the Public Security Bureau, drug rehabilitation centers, physical examination centers, physical examination offices of national conscription, etc. Drug tests are diverse and frequent. There is a huge market demand for drug test urine cups that can automatically separate the remaining samples from test samples. At present, after drug test urine cups on the market test samples, the samples therein will be contaminated by detection reagents and cannot be used for second determination test, as described in U.S. Patent 7300633.

Although test samples can be separated from collected samples in a conventional technology, such operation is costly and difficult. For example, U.S. Patent No.7300633 describes a piston urine cup; when the piston thereof is pushed forward, a liquid sample in a collection chamber, such as urine, is transferred from the collection chamber to the testing chamber; a testing element for testing an analyte in the liquid sample is provided in the testing chamber; and the liquid sample in the collection chamber is isolated by the piston, such that liquid samples from two places will not be confused, facilitating subsequent determination test. Although the test sample can be separated from the collected sample in this way, the piston urine cup is costly and difficult to operate. After all, it takes a lot of effort to push the piston. Because the piston needs to transfer the sample, the wall of the piston needs to have the effect of liquid sealing. To achieve the sealing effect, the piston and the piston chamber need to be closely combined.

For another example, U.S. Patent No. 8992855 describes a device for collecting a liquid sample, and the device includes a piston structure that is integrated with the cover and moves together with the cover. Although the test samples can be separated from the collected samples, great pressure needs to be overcome before the test samples enter into the testing chamber, and the dimensions of the cover and the cup mouth need to be precisely designed, such that the piston integrated with the cover can be accurately inserted into a separation chamber.

In addition, after the primary tests of these traditional collection and testing devices are completed, if subsequent determination tests thereof need to be performed, the whole collection and testing devices need to be transported to the determination testing agencies for further determination test. This will bring many problems, including at least the following problems: firstly, most liquid collection and testing devices are only provided with primary testing chambers at present. If the subsequent determination tests need to be performed, only the whole device including urine and test strips can be delivered to the determination testing agencies for detection. The samples in the urine cup may be contaminated by the detection reagent. Secondly, when the whole device is delivered to the determination testing agencies, because the cup mouth is large, there is a risk of liquid leakage during transportation, which requires more cost to make the device have a better sealing effect to minimize the risk of leakage. Thirdly, the determination testing agencies require a huge low-temperature warehouse to store the whole testing device, to prevent the liquid sample from deteriorating and prepare for the possible further determination test, which causes the cost of the determination testing agencies (which can be called the secondary testing agencies) to increase greatly.

In view of the above technical problems, it is necessary to improve them and provide an alternative approach to solve the drawbacks of the prior art.

### BRIEF SUMMARY OF THE INVENTION

In view of the above situations, in order to overcome the defects of the prior art, the purpose of the present invention is to provide a testing device capable of separating a primary test sample from a determined test sample. After collecting a liquid sample, the testing device can allow the primary test sample and the determined test sample to enter two chambers before or after test, for example, a first chamber and a second chamber. Then, after or before the primary test, the second chamber can be separated from a primary sample collection chamber (the first chamber), such that detachable separation is made between the second chamber and the primary sample collection chamber.

On the one hand, the present invention provides a device for collecting a liquid sample, and the device includes: a first chamber for collecting the liquid sample and a second chamber for collecting a liquid sample for determination test; where the first chamber is detachably combined or connected with the second chamber.

In some preferred embodiments, before the second chamber is not separated from the first chamber, the first chamber is in fluid communication with the second chamber; alternatively, when the first chamber and the second chamber are combined together, the first chamber is in fluid communication with the second chamber. In some preferred embodiments, after the second chamber is separated from the first chamber, the first chamber is not in fluid communication with the second chamber. In some preferred embodiments, before the second chamber is separated from the first chamber, the first chamber is in fluid communication with the second chamber. In some preferred embodiments, after the second chamber is separated from the first chamber, the liquid sample from the first chamber is stored in the second chamber. In some preferred embodiments, when or after the first chamber collects the liquid sample, the second chamber also collects the liquid sample from the first chamber. In some preferred embodiments, when the first chamber collects the liquid sample, the second chamber also collects the liquid sample from the first chamber. In some embodiments, the first chamber includes a testing element therein, and the testing element is used for initially testing an analyte in the liquid sample.

In some other embodiments, the first chamber and the second chamber are combined detachably or separated through combination positions. Alternatively, the first chamber and the second chamber can be directly combined together or indirectly combined together through a specified structure. The combination position may be a place where the first chamber and the second chamber are in physical contact. In some embodiments, the second chamber includes a chamber and an opening, where the opening is used for receiving liquid from the first chamber and allowing the liquid to flow into the second chamber.

In some embodiments, an external screw thread is provided outside the opening of the second chamber; the bottom of the first chamber is provided with a channel or a hole with an internal screw thread; and the external screw thread of the second chamber and the internal screw thread of the bottom of the first chamber are mutually meshed, such that the second chamber and the first chamber are connected together. In some embodiments, the device further includes a cover for sealing the second chamber, where the cover includes a screw thread, and the screw thread of the cover is meshed with the external screw thread of the second chamber to seal the second chamber.

In some embodiments, the second chamber is detachably combined or connected with the first chamber through the base; alternatively, the base is detachably connected with the first chamber; when the base needs to be separated from the second chamber, the base is separated from the first chamber, thereby driving the second chamber to depart from the first chamber. In some embodiments, the cover for sealing the second chamber is provided on the base; and when the base departs from the first chamber, the cover is removed from the base to seal the second chamber. In some embodiments, the first chamber is used for receiving the liquid sample; and when the liquid sample flows into the first chamber, a part of the liquid sample is used for testing and assaying and the other part thereof flows into the second chamber. In this case, the second chamber is fixedly connected with the base, such that the base departs from the first chamber, driving the second chamber to depart from the first chamber. Of course, it is understood that in some embodiments, the second chamber is connected with the first chamber such that the base is indirectly combined with the first chamber.

In some embodiments, the first chamber has a structure where a collector is allowed to release the liquid sample; and when the collector is inserted into the structure, the collection head of the collector can be extruded to release the liquid sample. In some embodiments, an extrusion table for releasing the liquid sample is provided at the bottom of the first chamber; the extrusion table is used for contacting with the absorption element of the liquid sample collector; and when the absorption element fits with the extrusion table, the absorption element absorbing the liquid sample is allowed to be compressed to release the liquid sample onto the extrusion table.

In some embodiments, the extrusion table is in fluid communication with the second chamber; therefore, when the liquid sample is present in the extrusion table, the part of the liquid sample flows into the second chamber, while the other part thereof is retained in the first chamber for primary test. In some embodiments, the first chamber includes a channel that is in fluid communication with the extrusion table and the second chamber. In some embodiments, the channel includes an inlet and an outlet; the extrusion table is provided with the inlet; the bottom of the first chamber is provided with the outlet; then, the channel is connected with the opening of the second chamber. When the absorption element is compressed on the extrusion table, the part of the liquid sample flows into the channel and then flows into the second chamber through the outlet for subsequent secondary determination test.

In some embodiments, the base and the second chamber are of an integral structure; the base is provided with a first surface contacting with the bottom of the first chamber; the opening of the second chamber is higher than the first surface; there is a distance between a highest end of the opening and the first surface, and a neck-shaped pipe of the second chamber is within the distance (in some embodiments, the opening and the neck-shaped pipe form the second chamber); the screw thread is provided outside the neck-shaped pipe and fits with the screw thread of the hole in the bottom of the first chamber, such that the second chamber is in fluid communication with the first chamber and the base is indirectly connected with the bottom of the second chamber through the screw thread of the neck-shaped pipe of the second chamber. When the screw threads fit with each other, for convenience, the neck-shaped pipe can be allowed to align at the outlet of the channel of the first chamber and the base rotates; when the screw threads are used up and the base cannot rotate again, the base fits with the bottom of the first chamber; therefore, viewed from the outside, the device is an integral testing device, and the base and the bottom of the first chamber are not misaligned. In such an arrangement, the neck-shaped pipe has an opening and an internal chamber, wherein the internal chamber can accommodate a specified amount of liquid, such as 1 µL to 10 ml, or 5 ml. When the liquid exists in the second chamber, the base rotates reversely again such that the second chamber is separated from the first chamber and the base is separated from the bottom of the first chamber; and then the cover is used for sealing the opening of the second chamber; specifically, the cover includes the internal screw thread, the internal screw thread fits with that outside the neck-shaped pipe of the first chamber, and then the base is transported to a laboratory center for secondary test or secondary determination test. If it is desired that this way is implemented, the base is relatively high, so the device is easy to rotate by hands and is also easy to separate from the first chamber through rotation. If the base is not high, the base is inconveniently held by fingers such that the base is difficult to separate from the first chamber through rotation. This specific embodiment is a specific embodiment where the second chamber is separated from the first chamber.

In some embodiments, because the base is relatively low and the base is kept consistent with the overall shape of the chamber before separated from the first chamber, a boundary between the base and the chamber is difficult to distinguish from the appearance. In such a way, the base is difficult to separate from the first chamber through the ideal rotation thereof by hands. This is due to a fact that such operation is inconvenient and relatively difficult and that the improper operation may cause the leakage of the liquid in the second chamber to pollute an operator, and the liquid in the second chamber can also leak from the channel to the outside. Therefore, in order to facilitate the operation by the operator, another convenient structure is provided such that the base is easy to separate from the first chamber. Therefore, in some embodiments, a traction mechanism is provided on the base and can apply a traction force to the base, such that the base is separated from the first chamber, driving the second chamber to be separated from the first chamber.

Initially, a traction mechanism may be fixedly connected with the base or may be assembled at any position of the base. When necessary, the traction mechanism is removed from the base and the traction mechanism is combined with the base, an external force is applied to the traction mechanism to separate the base from the first chamber. When the traction mechanism is initially connected with the base, the traction mechanism is directly pulled to separate the base from the traction mechanism, thereby driving the second chamber to depart from the first chamber. In this case, the neck-shaped pipe of the first chamber is not provided with the screw thread, and the neck-shaped pipe is connected with the outlet of the channel of the first chamber through a piston, such that the base can depart from the first chamber through traction, driving the second chamber to be separated from the outlet of the channel to separate the second chamber from the first chamber. The traction mechanism may be a pulling ring directly connected with the base.

When the traction mechanism is separately assembled on the base and the second chamber is also equipped on the base, the second chamber fits with the screw thread at the outlet of the channel of the first chamber through a first screw thread of the neck-shaped pipe, such that the base and the second chamber are connected as a whole. When the second chamber needs to be removed, the bottom of the second chamber is provided with a second screw thread and the screw thread is provided on the traction mechanism; the screw thread on the traction mechanism fits with the second screw thread of the second chamber, such that the traction mechanism is connected with the second chamber. Then, the traction mechanism continues to rotate to drive the second chamber to rotate on the base in a direction opposite to the direction in which the base is connected to the first chamber, such that the second chamber is separated from the first chamber, and the base is separated from the first chamber. After the separation, a cover can be used for sealing the opening of the second chamber in such a way that a plug is provided on the cover and directly inserted into the opening of the second chamber. A special arrangement needs to be made in the screw thread arrangement direction, to be specific, if the screw thread of the neck-shaped pipe of the second chamber is fixed with the outlet of the channel of the first chamber through clockwise rotation, the screw thread of the neck-shaped pipe of the second chamber is separated from the outlet of the channel of the first chamber through counterclockwise rotation; therefore, when the traction mechanism and the second chamber perform screw thread rotation, the screw thread is fixed with the second chamber through counterclockwise rotation; after fixed screw threads are used up, the traction mechanism continues to rotate counterclockwise to drive the second chamber to rotate counterclockwise. Of course, accordingly, if the screw thread of the second chamber is fixed with the outlet of the channel through counterclockwise rotation, the screw thread of the second chamber is separated from the outlet of the channel through clockwise rotation; therefore, when the traction mechanism and the second chamber perform screw thread rotation, the screw thread is fixed with the second chamber through clockwise rotation; after fixed screw threads are used up, the traction mechanism continues to rotate clockwise to drive the second chamber to rotate counterclockwise. In this embodiment, initially, the traction mechanism can be directly and fixedly with the second chamber, and the rotation of the traction mechanism can directly drive the second chamber to rotate, such that the second chamber can be separated from the first chamber, driving the base to be separated from the first chamber.

In other some embodiments, the base is provided with a small convex chamber thereon; the small chamber is provided with the external screw thread fitting with the screw thread of the outlet of the channel; the bottom of the small chamber is provided with one plug, such that the small chamber and the plug at the bottom thereof form the second chamber; in this case, the rotation of the base enables the small chamber to depart from the first chamber, the small chamber is actually integrated with the base as a whole, and the base is allowed to depart from the small chamber through rotation thereof, thereby driving the second chamber thereon to depart from the first chamber. Of course, one chamber may be directly provided on the base and has a protruding neck-shaped pipe that is inserted into the opening of the channel of the first chamber in the form of the piston; the bottom of the base is provided with the traction mechanism; the base is allowed to depart from the first chamber through the traction mechanism, whereby driving the second chamber thereon to depart from the base.

In some embodiments, in order to reduce the overall volume, two spaces are provided on the base, where one space is used for placing the cover, and the other space is used for storing the second chamber. In some embodiments, the base includes two surfaces, namely, a front surface and a back surface or a first surface and a second surface, where the first surface faces the bottom of the first chamber, and the back surface or the second surface is the other surface. When the base is connected with the first chamber, the back surface or the second surface is the bottom of the whole testing device. In some embodiments, the front surface is provided with one recess area; the cover is provided in the recess area; a platform area is provided next to the recess area; the second chamber is provided in the platform area; and the opening of the second chamber is higher than a planar area; therefore, when the front surface of the base is allowed to contact with the bottom of the first chamber, the opening of the second chamber is allowed to be butted with the outlet of the channel at the bottom of the first chamber, such that the first chamber is in fluid communication with the second chamber, the base fits with the second chamber, and the base is used as the base of the second chamber, helping the testing device erect on the table.

In some embodiments, the second chamber and the base are integrally formed. Of course, the second chamber and the base can be fixedly connected together, and the second chamber can also be separated from the base and assembled on the base when necessary. In some embodiments, the bottom of the second chamber has a pin hole, and a pin element is allowed to be inserted into the pin hole, such that the second chamber is connected with the first chamber; in this embodiment, the pin element is a part of the traction mechanism. In some embodiments, a pin is connected with the pulling ring, and the second chamber can be separated from the first chamber through the pulling ring. Because the second chamber is connected with the base, the second chamber and the first chamber, as well as the base and the first chamber can be separated through the pulling ring, where the pulling ring and the pin element together form the traction mechanism. In some embodiments, the pin is connected with the pulling ring and provided with the screw thread thereon, and the pin hole is also provided with the screw thread, such that the pin with the pulling ring is connected with a threaded hole in the bottom of the second chamber through meshing of the screw threads. Of course, in some embodiments, one pulling ring is integrally formed at the bottom of the second chamber. With the pulling ring being at an initial position, the pulling ring is located at the bottom of the base and fixed parallel to the ground; besides, the pulling ring is not higher than the base and hidden in the base. When necessary, the pulling ring is allowed to be perpendicular to the ground surface of the second chamber or the ground surface of the base, and the pulling ring is pulled such that the base is separated from the bottom of the first chamber. The "pulling ring" here can also be of any other structure, and any traction structures through which the base can be separated from the first chamber are desirable; for example, soft ropes, soft plastic tapes, or pulling rings that are similar to those of "ring-pull cans" in design can be used in specific embodiments of the present invention.

The pulling ring or the traction mechanism being directly connected with the second chamber has the advantage of ensuring the second chamber to smoothly depart from the first chamber. If the traction mechanism or the pulling ring is not directly connected with the second chamber, but is connected with other positions of the base, the base can depart from the first chamber when the pulling ring is pulled, without ensuring that the second chamber is easy to depart from the first chamber. This is due to a fact that the connection of the base and the first chamber depends on fitting of the opening of the second chamber and the hole in the first chamber, such that the pulling ring is directly connected with the second chamber. When the pulling ring is pulled, an acting force is directly applied to the second chamber such that the second chamber is separated from the first chamber and then fixed onto the base, driving the base to be separated from the bottom of the first chamber. Otherwise, the acting force is used to pull other parts of the base, instead of being directly applied to the second chamber. This may cause a part of the base to be separated from the first chamber, and the neck-shaped pipe of the second chamber is still located in the channel of the first chamber; or improper force may cause the neck-shaped pipe of the second chamber to be broken and remain in the channel of the first chamber, such that the second chamber cannot be sealed by the cover, resulting in failure to take secondary determination samples. This is due to a fact that in some cases, the pipe does not need to have too large space, after all, and too many determined samples are not required, which is easy to cause fracture.

In some embodiments, the first chamber is provided with a carrier therein, and the testing element is provided on the carrier and used for testing the analyte in the liquid sample located in the first chamber for primary test. The carrier is located in the first chamber and between the extrusion table and a side wall of the first chamber. In some embodiments, a sample application area of the testing element is located at the bottom of the first chamber. In some embodiments, the extrusion table and the side wall of first chamber form a collection tank, and the liquid released from the extrusion table flows into the collection tank and contacts with the testing element in the collection tank to complete the primary test.

On the other hand, the present invention provides a method for testing an analyte in a sample, and the method includes: providing the testing device as described above, where the device includes a first chamber for receiving a test sample and a second chamber for secondary determination test; the first chamber is used for collecting the test sample; a part of the test sample is allowed to remain in the first chamber and the other part thereof is allowed to flow into the second chamber for retention; after the test, the second chamber is allowed to be separated from the first chamber.

In some embodiments, the first chamber has a channel that is in fluid communication with the first chamber and the second chamber, and the second chamber has an opening, and the opening of the second chamber is located at the outlet of the channel, such that the first chamber is detachably combined with the second chamber. In some embodiments, the second chamber is located on a base; a traction mechanism is provided on the base; after the primary test, a force is applied to the traction mechanism, such that the base is separated from the first chamber, driving the second chamber to be separated from the first chamber. In some embodiments, the base is connected to the first chamber through the second chamber to form a detachable combination. In some embodiments, the traction mechanism is directly connected with the second chamber, and such connection may be fixed connection or detachable connection. In some embodiments, when the second chamber or the second chamber with the base departs from the first chamber, the opening of the second chamber is sealed with the cover, and the subsequent secondary determination test is performed.

In some embodiments, the traction mechanism and the cover are located on the base, but not located in the first chamber. In some embodiments, the base has a space for accommodating the cover, and the traction mechanism is provided on the base. The traction mechanism has a pulling ring, a testing element or pin connected with the base. The pulling ring is pulled, such that the second chamber is separated from the first chamber, driving the base to be separated from the first chamber.

### Beneficial effect

The above structure is simple and reasonable, has low cost of used materials and excellent performance, and is convenient for secondary test. Especially, when the subsequent determination test needs to be performed, the whole testing device does not need to be delivered to the testing agencies for detection, but only the second chamber is removed from the device and then delivered to the testing agencies. Such operation is not only safe but space-saving, cost-saving and more environment-friendly.

### BRIEF DESCRIPTION OF THE DRAWINGS

FIG. 1 is a schematic diagram showing a three-dimensional structure of an integrally assembled testing device according to a specific embodiment of the present invention.
FIG. 2 is an exploded schematic diagram of a testing device according to a specific embodiment of the present invention, that is, an exploded diagram of a structure where a base is separated from a first chamber.
FIG. 3 is a schematic diagram showing an exploded three-dimensional structure of a base and a first chamber according to a specific embodiment of the present invention.
FIG. 4 is a schematic diagram showing a longitudinal cross-section structure of combination of a first chamber and a base.
FIG. 5 is a schematic diagram showing a cross-sectional structure of a first chamber.
FIG. 6 is a schematic diagram showing a schematic diagram of a first chamber in a direction looking up from the bottom thereof.
FIG. 7 is a top view of a base with a cover and an opening of a second chamber.
FIG. 8 is a schematic diagram showing a three-dimensional structure of a base according to a specific embodiment of the present invention, where an opening and a neck-shaped pipe are directly connected with the base as a whole, and the base or a plug is located below the opening.
FIG. 9 is a schematic diagram showing a structure where a base cover is removed to seal an opening of a second chamber.
FIG. 10 is a schematic diagram showing a three-dimensional structure according to another embodiment of the present invention.
FIG. 11 is a schematic diagram showing a three-dimensional structure according to another specific embodiment of the present invention (a second chamber is located on a side wall of a first chamber and a cover is also located on the first chamber).
FIG. 12 is a schematic diagram showing a cross-sectional structure according to another embodiment of the present invention.

### DETAILED DESCRIPTION OF THE INVENTION

The following further describes the structures involved in the present invention or the technical terms used therein. Unless otherwise specified, they shall be understood and explained according to the general terms commonly used in the prior art.

### Detection

Detection means to assay or detect presence or absence of a substance or material, including but not limited to, a chemical substance, an organic compound, an inorganic compound, a metabolite, a drug, a drug metabolite, an organic tissue, a metabolite of an organic tissue, a nucleic acid, a protein or a polymer. In addition, detection means that the amount of a substance or material is tested. Further, assay also means immunoassay, chemical assay, enzyme assay, and the like.

### Samples

Samples or specimens that can be detected by the testing device of the present invention or collected by the collector (omitted) of the present invention include biological liquids (for example, case liquids or clinical samples). Liquid samples or fluid samples may be derived from solid or semi-solid samples, including feces, biological tissues and food samples. The solid or semi-solid specimens may be converted to liquid specimens by any appropriate methods, such as mixing, mashing, macerating, incubating, dissolving, or digesting the solid specimens by enzymolysis in suitable solutions, such as water, phosphate solutions, or other buffer solutions. "Biological samples" include animal, plant, and food derived samples, including, for example, human or animal derived urine, saliva, blood and components thereof, spinal fluid, vaginal secretions, sperm, feces, sweat, secretions, tissues, organs, tumors, cultures of tissues and organs, cell cultures, and media. Preferably, the biological sample is urine; and preferably, the biological sample is saliva, sputum, nasal secretion, or the like. Food samples include food processing substances, final products, meat, cheese, wine, milk, and drinking water. Plant specimens include specimens derived from any plants, plant tissues, plant cell cultures, and media. "Environmental specimens" include specimens derived from the environment (for example, liquid specimens from lakes or other bodies of water, sewage specimens, soil specimens, groundwater, seawater, and waste liquid specimens). The environmental specimens may further include sewage or other waste water.

An appropriate testing element according to the invention can be used to detect any analyte. Preferably, the testing device of the present invention is used to detect small drug molecules in saliva and urine. Preferably, the testing device can be used to detect small molecular substances such as viruses and bacteria in saliva, throat or nasal fluid. Any samples of the above forms may be collected using a collector (omitted) according to the present invention, regardless of being solid or liquid at the beginning, provided that these liquids or liquid samples can be absorbed by an absorption element generally located on the collector. Generally, the absorption elements here are made of absorbent materials, dry at the beginning, and can absorb liquid samples or fluid samples through the capillary or other characteristics of the absorption element materials, such that the fluid samples can be kept in the absorption elements. The absorption material may be any material capable of absorbing liquid, such as sponge, filter paper, polyester fiber, gel, non-woven fabric, cotton, polyester film, yarn, and flocking. When flocking swabs are used, flocking swabs as described in the following patents can be used as a part of the present invention: US 8,114,027, US 8,317,728, US 8,979,784, US 9,011,358, US 9,173,779, US 10,327,741, AU 2004226798, JP 4579902, and ZL 200610099310.9. In some embodiments, the dry absorption element is hard; for example, the wet sponge becomes soft, and then can be compressed, thus releasing liquid. Of course, a sparse sponge, such as a sponge swab, can also absorb liquid samples, having a small amount of absorbed liquid samples, such as 5-100 µL.

Of course, the absorption element may be made of a water absorbent material or a non-water absorbent material. However, the absorption element is provided with holes, screw threads, and caves on which the samples can be collected. Generally, the samples are solid or semi-solid samples, and filled between screw threads and in the holes or caves for collection. Of course, optionally, the absorption element may be composed of some non-absorbent fibers and hairs, and these materials are used to scrape a solid, semi-solid or liquid sample, such that these samples can be retained on the absorption element. Samples that can be tested by the testing device of the present invention include biological liquids (for example, case liquids or clinical samples). Liquid samples or liquid specimens may be derived from solid or semi-solid samples, including feces, biological tissues and food samples. The solid or semi-solid samples may be converted to liquid samples by any appropriate methods.

An appropriate testing element according to the present invention can be used to test any analyte. Preferably, the testing device of the present invention is used to detect small drug molecules in saliva and urine. Of course, the collection device of the present invention can be used for collecting any of the above samples, regardless of whether the samples are solid or liquid at the beginning. Provided that these liquids or liquid samples flow into the first chamber, these liquid samples can flow into the second chamber at the same time or later. Because the second chamber is detachably combined or connected with the first chamber, the second chamber is allowed to be separated from the first chamber when the subsequent determination test needs to be performed; therefore, the liquid in the second chamber can be performed for the secondary test while the liquid in the first chamber can be performed for the primary test. Optionally, the liquid in the second chamber can be performed for the primary test, and the liquid in the first chamber can be performed for the secondary test.

### Downstream and upstream

Downstream or upstream is divided according to a flow direction of a liquid. Generally, a liquid flows to a downstream area from an upstream area. The downstream area receives the liquid from the upstream area, and a liquid also may flow to the downstream area along the upstream area. Here, downstream or upstream is generally divided according to a flow direction of a liquid, for example, on some materials where capillary force is utilized to promote the flow of a liquid, a liquid may overcome gravity to flow towards an opposite direction to the gravity; and in this case, downstream or upstream is divided according to a flow direction of the liquid. For example, in the collection device of the present invention, in some preferred embodiments, the first chamber serves as a chamber for collecting a liquid sample, while the second chamber is in fluid communication with the first chamber, and the liquid flowing into the first chamber flows into the second chamber. The first chamber can be called upstream while the second chamber can be called downstream. Of course, such flow is natural flow of liquid under the action of gravity. Optionally, such natural flow is the flow of the liquid from the first chamber to the second chamber.

### Gas communication or liquid communication

Gas communication or liquid communication means that liquid or gas can flow from one place to another. In the flow process, the liquid or gas may pass through some physical structures that play a guiding role. The "pass through some physical structures" here means that the liquid passes through the surface of these physical structures or the internal space of these physical structures and flows to another place passively or actively, where the passive flow is usually caused by external forces, such as the flow under the capillary action. The flow here may also be a flow due to self-action (gravity or pressure) of the liquid or gas, and also may be a passive flow.

### Detachable combination

A detachable combination means that two components are connected in several different states or positional relationships. For example, with two components being physical components, they can be separated at the beginning and then connected or combined in an appropriate first case, and separated in an appropriate second case. Physically, such separation is spatial separation without contact. Alternatively, the two components are combined at the beginning, and can be physically separated from each other when appropriate. In short, combination or separation of two components or two objects can be easily made and repeated many times. Of course, the combination or separation can also be single-use. In addition, such combination can be a detachable combination between two components, or a two-by-two detachable combination between three or more components. For example, a first component, a second component, and a third component are provided, where a detachable combination is made between the first component and the second component or between the second component and the third component.

### Testing element

The testing element can be a lateral flow test strip that can detect a variety of analytes. Of course, other appropriate testing elements also can be used in the present invention, and an element that can be used to detect whether a sample or a specimen contains an interested analyte may be called as the testing element. Such testing can be based on any technical principles, such as immunology, chemistry, electricity, optics, and physics. Various testing elements can be combined for use in the invention. One form of the testing elements is a test strip.

Test strips used in the present invention may be commonly referred as lateral flow test strips. The specific structure and testing principle of the test strips are well known to a person skilled in the art in the prior art. A common test strip includes a sample collection area or a sample application area, a label area, a testing area and a water absorption area. The sample collection area includes a sample receiving pad, the label area includes a label pad, and the water absorption area may include a water absorbent pad. The testing area includes necessary chemical substances for detecting the presence or absence of the analyte, such as immunoreagents or enzyme chemical reagents. The nitrocellulose membrane test strip is commonly used, that is, the testing area includes a nitrocellulose membrane, and a specific binding molecule is immobilized on the nitrocellulose membrane to display the testing result; and other test strips such as cellulose acetate membrane or nylon membrane test strips may also be used. Of course, in the downstream of the testing area, there may also be a testing result control area. Generally, the control area and the testing area in the form of horizontal lines, namely, a test line or a control line. Such test strips are conventional. Of course, they may also be other types of test strips for detection under the capillary action. In addition, there are dry chemical reagent components on common test strips, for example, an immobilized antibody or other reagents. When the test strip contacts a liquid, the liquid flows along the test strip under the capillary action, and the dry reagent components are dissolved in the liquid and treated in a next area, and the dry reagents react in the area for necessary detection. The liquid flow mainly relies on the capillary action. These testing elements are described and recorded in the following documents: Li Fugang, Study on the Regeneration of Nitrocellulose Membrane and Its Protein Adsorption Capability; Ma Hongyan, Li Qiang, et al., Analysis on the Properties of Chromatographic Membrane Materials in Colloidal Gold Diagnostic Kit; Wang Yong, Wang Luhai, et al., A New Colloidal Gold Immunochromatographic Test Strip. Here, all of the test strips can be applied to the test device of the present invention or can be disposed in contact with the liquid samples in a testing chamber or used to detect the presence or absence of an analyte in the liquid samples that enter a testing chamber, or the quantity thereof.

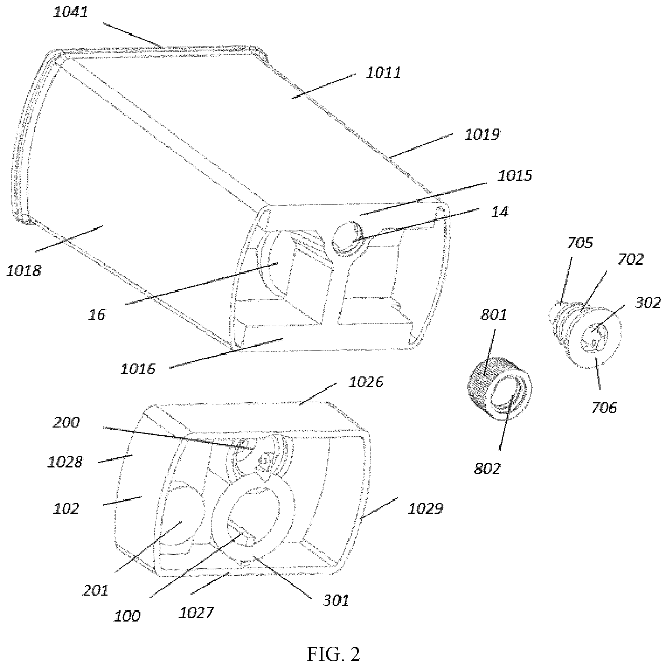

Except that the foregoing test strip that contacts the liquid sample of the first chamber itself to test whether the liquid samples contain analytes. In some preferred embodiments, the testing element can also be provided on a card with a plurality of grooves, and the testing element is located in the grooves, and the whole test strip is provided in the first chamber 101, such that the sample application area of the testing element is located at the bottom of the testing chamber to contact with the liquid sample. The liquid sample can be obtained from the first chamber 101, and the sample application area of the testing element is located in the sample collection area of the testing chamber to contact with the liquid sample, thereby implementing the test of the analyte. The testing chambers and the carriers or the test strips that are included in the testing chambers are described in detail in U.S. Patent No. US9,925,539B2 of the applicant, and all specific embodiments can be realized as a specific embodiment of the present invention. For example, in FIG. 2 of the U.S. Patent, 4 denotes the carrier, and 2 denotes the testing element; the carrier and the testing element are respectively provided on bilateral planes of the testing chamber 11. The collector 14 is used for absorbing liquid, and the tail end of the collector is provided with an absorption element for absorbing liquid samples. When testing needs to be performed, the collector is inserted into the testing chamber 11, and the absorption element is extruded to release the liquid, thereby implementing the test of the analyte in the liquid sample.

For example, in some embodiments, the liquid sample can be collected in the first chamber, then the test strip or the card or carrier with the test strip is inserted into the first chamber for testing, and finally a part of the liquid flows into the second chamber. After the test in the first chamber is implemented, the second chamber is separated from the first chamber, and the second chamber is left for secondary determination test. It can be understood by those skilled in the art that according to the present invention, these test strips may or may not be provided on the carrier.

### Analyte

Examples that can use an analyte related to the invention include some small-molecule substances, including drugs (such as drug of abuse). "Drug of Abuse" (DOA) refers to the use of a drug (typically functions to paralyze the nerves) not directed to a medical purpose. Abuse of these drugs will lead to physical and mental damage, dependency, addiction and/or death. Examples of drug abuse include cocaine; amphetamine (AMP) (e.g., Black Beauty, white amphetamine tablets, dexamphetamine, dexamphetamine tablets, and Beans); methamphetamine (MET) (crank, meth, crystal and speed); barbiturate (BAR) (such as Valium, Roche Pharmaceuticals, Nutley, and New Jersey); sedatives (i.e., a sleep aid medicine); lysergic acid diethylamine (LSD); inhibitors (downers, goofballs, barbs, blue devils, yellow jackets, and methaqualone); tricyclic antidepressants (TCAs, i.e. imipramine, amitriptyline, and doxepin); dimethylenedioxymethylaniline (MDMA); phencyclidine (PCP); tetrahydrocannabinol (THC, pot, dope, hash, weed, etc.); opiates (i.e., morphine (MOP) or opium, cocaine (COC), heroin, and hydroxydihydrocodeinone); and anxiolytic drugs and sedative-hypnotic drugs. The anxiolytic drugs are mainly used for relieving anxiety, tension, and fear, and stabilizing emotion, and have hypnotic and sedative effects. The anxiolytic drugs include benzodiazepines (BZO), atypical benzodiazepines (BZ), fused dinitrogen NB23C, benzodiazepines, ligands of BZ receptors, open-ring BZ, diphenylmethane derivatives, piperazine carboxylates, piperidine carboxylates, quinazolinones, thiazine and thiazole derivatives, other heterocycles, imidazole-type sedative/analgesic drugs (e.g., oxycodone (OXY) and methadone (MTD)), propylene glycol derivatives-carbamates, aliphatic compounds, anthracene derivatives, and the like. The test device of the invention may also be used for detecting drugs belonging to a medical use but easy to be taken excessively, such as tricyclic antidepressants (imipramine or analogues) and acetaminophen. These drugs are metabolized into micromolecular substances after absorbed by human body. These micromolecular substances exist in blood, urine, saliva, sweat and other body fluids or in some body fluids.

For example, the analyte detected by the present invention includes but is not limited to creatinine, bilirubin, nitrite, (nonspecific) proteins, hormones (for example, human chorionic gonadotropin, progesterone, follicle-stimulating hormone, etc.), blood, leucocytes, sugar, heavy metals or toxins, bacterial substances (such as proteins or carbohydrates against specific bacteria, for example, *Escherichia coli* 0157:H7, *Staphylococcus, Salmonella, Fusiformis, Camyplobacter genus, L. monocytogenes, Vibrio,* or *Bacillus cereus*) and substances related with physiological features in a urine sample, such as pH and specific gravity. Chemical analysis of any other clinical urine may be performed by lateral flow detection in combination with the device of the present invention.

### Primary test and secondary determination test

Primary test here means initial or first test of samples. In some embodiments, generally, the initial test is a primary test, and the testing results are not very accurate; or the initial test is just a rough test. Generally, if the concentration of the analyte is very high or low, it can be distinguished through the primary test; and when the concentration is approximate to the threshold of the primary test, it cannot be well identified. In this case, it is hoped that the same sample will be tested more accurately, and such a testing method has the accuracy or sensitivity higher than the primary test method. For example, the primary test is performed by lateral flow immunoassay, and the secondary test can be performed by more accurate methods, such as chemiluminescent immunoassay, ELSA method and liquid chromatography-mass spectrometry. Of course, the secondary test here can also share a way with the primary test. For example, some samples can be tested in the primary test way, and the other samples can be tested in a same way as the primary test way after a period of time. For example, such a way can be used when the storage performance of the samples is detected.

### Testing device or collection device

The testing device refers to a device for detecting the presence or absence of an analyte in a sample. The collection device refers to a device for collecting and storing liquid samples. The testing device can include a collection device, and the collection device can also include the testing device, or the collection device is separated from the testing device; during the test, the collection device and the detection device are combined to complete the test. Alternatively, the collection device and the detection device are of an integral structure, and therefore the collected liquid sample can be tested immediately to obtain the testing result, and at the same time, the test sample can be separated from the collected sample, such that secondary test can be performed (if necessary). The testing device and the testing chamber here are interchangeable, and the collection device and the collection cavity are also interchangeable, but only their functions are interchanged depending on their different roles. For example, the collection device of the present invention may not include the testing chamber or the first chamber, but the collection device may include the testing element or the carrier with the testing element; the collection device including the testing element may also be called the testing device, for example, the first chamber includes the testing element. Of course, the collection device can include a space where the testing element is provided, but does not necessarily include the testing element, and the testing element can be combined with the collection device at any suitable time to serve as the testing device. For example, the collection device may include a space for accommodating the testing element, such as the testing chamber; alternatively, the liquid collection chamber of the collection device has a suitable position where the testing element or the carrier including the testing element is provided.

### Detachable combination of first chamber and second chamber

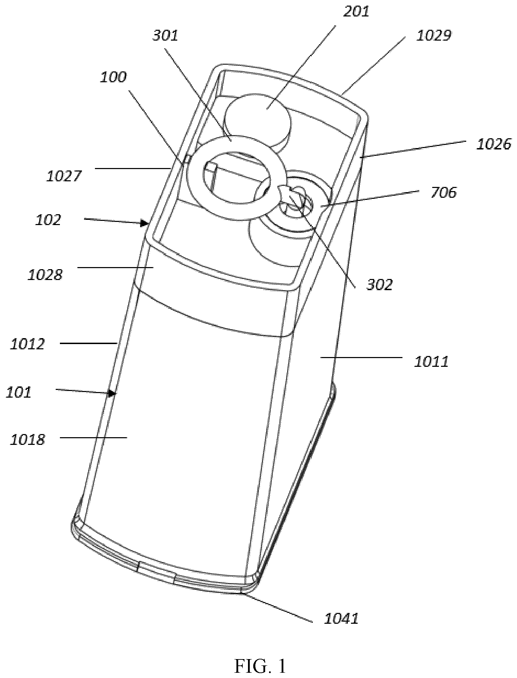

In some specific embodiments of the present invention, as shown in FIG. 1 - FIG. 9, the present invention provides a testing device for detecting the presence or absence of an analyte in a liquid sample or a device for collecting a liquid sample; the device includes a first chamber 101 and a second chamber; the first chamber 101 can be used as the collection chamber and used for collecting the liquid sample, where the first chamber is detachably combined or connected with the second chamber. Of course, the first chamber can be used as a location where the analyte in the liquid sample is subjected to the primary test. When used for the primary test, the first chamber 101 includes a lateral flow test strip therein, and the primary test is performed based on an immune method.

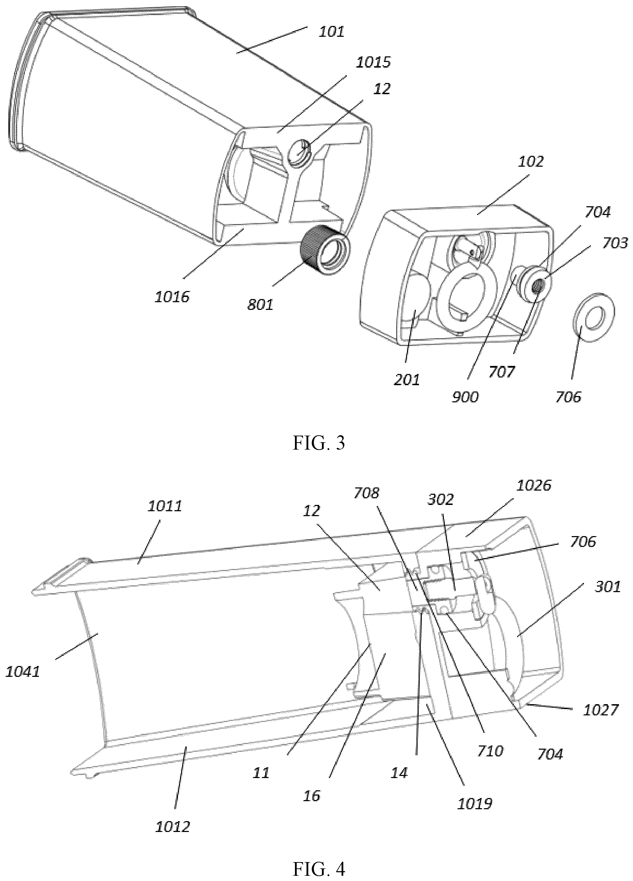

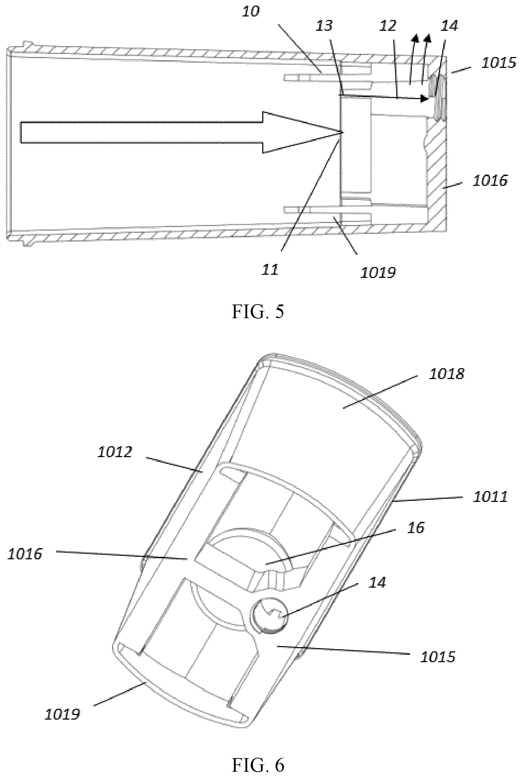

In some embodiments, the first chamber 101 is substantially a square structure with two opposite surfaces 1011, 1012. The two opposite surfaces also constitute the side walls of the first chamber, are relatively flat and wide, and are connected by two surfaces 1018, 1019, thereby enclosing an opening 1041. The first chamber further includes a bottom that is actually a plane. Because the bottom has an opening 14 of a channel 12, the bottom is divided into two parts 1016, 1015. Two grooves are formed in two relatively wide and flat surfaces 1011, 1012 at the bottom, and the test strip with the testing element can be placed in the grooves, the sample application area of the testing element is allowed to approach the grooves; the grooves are used for collecting the liquid sample; in the grooves, the sample application area of the testing element is in contact with the liquid sample for convenient testing; moreover, the test strip can lean against or be fixed onto two planes that are usually transparent; and the testing results of the test strip can be observed through the planes. One sample extrusion table 16 is provided at the middle bottom of the first chamber 101, and protrudes upward from the bottom of the first chamber and has a surface 11. When a sample collector (omitted, as shown by an arrow in FIG. 5) is inserted into the collection chamber, the absorption element of the collector (omitted) is in contact with the surface 11 of the extrusion table 16, and then a specified pressure is applied to the sample collector, such that the absorption element is compressed to release the liquid sample into the first chamber (as shown in FIG. 5 and FIG. 4). In this case, a part of the liquid sample is retained in the grooves formed by the extrusion table and the two surfaces 1011, 1012, and contacts with the sample application area of the test strip located in the grooves, thereby completing the primary test of the analyte. The channel 12 in direct communication with the second chamber 708 is provided in the extrusion table, such that the part of the liquid sample flowing from the extrusion table 11 flows into the second chamber 708, and the other part of the liquid sample flows into the grooves and contacts with the testing element. The channel 12 has an inlet 13 and an outlet 14. The inlet 13 is located on the surface 11 of the extrusion table (or the opening is located below the surface 11 of the extrusion table but in the middle of the extrusion table 12, and can also receive the liquid sample from the extrusion table), and the outlet 14 is located at the bottom of the first chamber (as shown in FIG. 5), while the second chamber is in direct communication with the outlet 14, such that the liquid from the extrusion table can flow into the second chamber 708 through the channel 12. The outlet is located on the bracket 1015 at the bottom of the first chamber and the bracket has a plane, facilitating contact of the bracket with the first surface of the base. The collector is typically used to collect saliva samples, of course, and may also be used to collect any other samples, where the collected samples are about 1-5 ml or 500-1000 µL; however, the samples for secondary determination test are about 1-100 µL, 1-10 µL, or 1-20 µL, so the mass spectrometry or more sophisticated device can be used for secondary test.

The second chamber has a chamber space used for accommodating the liquid sample for secondary determination test, and then also includes an opening 710, where the opening fits with the outlet of the channel 12 to receive the liquid from the channel 12. A neck-shaped pipe 709 is provided between the opening 710 and the chamber and has an external screw thread; an internal screw thread is provided inside the outlet 14 of the channel 12; and the second chamber can be connected with the first chamber in such a way that the external screw thread of the neck-shaped pipe is meshed with the internal screw thread inside the outlet of the channel. When there is the liquid sample in the second chamber, the second chamber can be removed from the bottom of the first chamber. If the second chamber is of a threaded structure, the second chamber is removed through reverse rotation, and the opening 710 of the second chamber is sealed with a cover 801 having a threaded structure 802 therein, and the second chamber is transported to a secondary determination testing agencies for testing. In this case, the liquid sample in the first chamber will not leak out of the channel 12. Generally, the extrusion table 16 has a sufficient height, and an amount of saliva or liquid collected is about 1-5 ml. The level of saliva distributed in the grooves on both sides of the extrusion table is unlikely to be beyond the extrusion table, thus avoiding the liquid in the first chamber from leaking out of the channel 12. Furthermore, once the liquid sample in the first chamber 101 is tested, the testing results for the testing element are scanned or photographed for saving before the second chamber departs from the first chamber. After the second chamber departs from the first chamber, the whole first chamber will be discarded or professionally treated as waste. In addition, the liquid samples flowing out of the extrusion table are directly divided into two parts of the liquid samples after the absorption element is extruded. After the two parts of the liquid samples are separated, they will not flow with each other and will not be confused. In a conventional test, some samples subjected to the primary test are performed for the secondary determination test. Because these samples contact with the testing element for the primary test, chemical substances on the testing element are dissolved into the test sample, causing potential impact on the test sample during the secondary test, which affects the accuracy and precision of the secondary test.

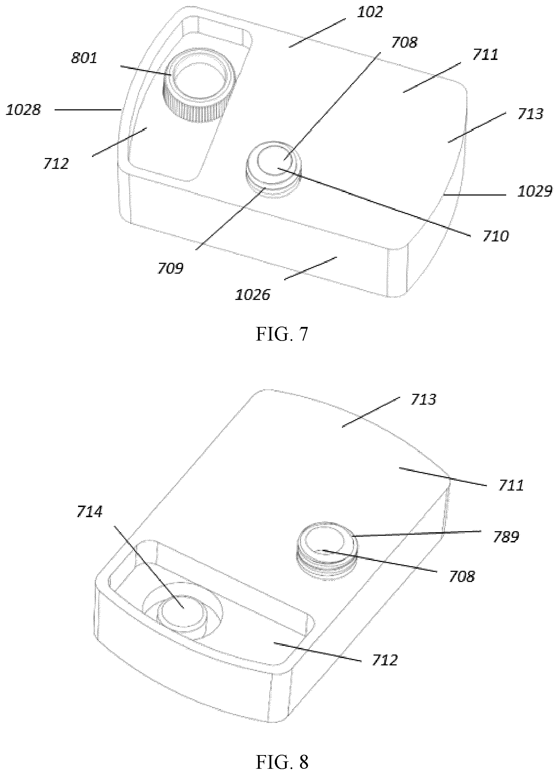

Because the second chamber is very small in volume and is located at the bottom of the first chamber alone, when the second chamber needs to depart from the first chamber, it is difficult to remove the second chamber from the first chamber. For example, the second chamber is rotated by fingers and is inconvenient to hold due to the small volume thereof. Generally, the second chamber may have a volume of 1 µL to 10 ml and is located at the bottom of the first chamber, thereby causing difficulty in directly removing the second chamber. Therefore, one base can be connected to the second chamber, such that the second chamber and the base can be connected together. It is more convenient and easier to realize separation of the second chamber and the first chamber through separation of the base and the first chamber. Therefore, in some embodiments, the second chamber is provided on the base 102, for example, the second chamber is located on a surface of the base, as shown in FIG. 7 and FIG. 8; when the bottom of the first chamber is a plane, the first surface of the base is also a plane structure and accordingly one small chamber 708 with an opening 710 protrudes from the first surface of the base; when the base is combined with the bottom of the first chamber, the opening 710 is allowed to align at the outlet 14 of the channel 12 of the first chamber, so the base is actually connected with the first chamber through the second chamber. The protruding small chamber 708 may be integrally formed with the base by injection molding or may be formed separately; and one hole is provided in the base such that the second chamber can be installed therein; in addition, the second chamber can be formed through combination of several parts. The following makes detailed explanations.

When the base is provided with the second chamber, it is desired that prior to delivery and installation, the second chamber fits with the first chamber in shape. Therefore, the base also has two wide surfaces 1026, 1027 and two narrow surfaces 1028, 1029; and the four surfaces constitute the whole base. During the specific installation, the wide surfaces 1026, 1027 are allowed to align at the wide surfaces 1011, 1012 of the first chamber respectively. From the outside, it seems that the wide surfaces 1011, 1012 of the first chamber extend downward. On the whole, this does not affect the coordination of the whole testing device. It can be easily understood that if the first chamber is circular, the base is also circular; and if the first chamber is elliptical, the base is also elliptical. In a word, it seems that the base 102 being combined with the first chamber 101 does not affect the coordination of the testing device, and from the outside, such an arrangement structure indicates the whole testing device. In order to distinguish the base from the first chamber, the outer surface of the base can be painted with color or can be injection molded with color. Generally, the surfaces 1011, 1012 of the first chamber need to be transparent, but the base may not be transparent, which increases the aesthetics of the testing device and facilitates the distinction between the base and the first chamber.

In some embodiments, the outer part of the second chamber provided on the base 102 has an external screw thread 709, where the second chamber protrudes upward from the first plane 711 of the base, and the protruding part thereof has the opening 710 and is a chamber space that can accommodate the liquid. When the base is assembled with the first chamber, the base is allowed to fit with the first chamber 101 in shape, and the opening 710 of the second chamber is allowed to align at the outlet of the channel 112 of the first chamber 101, and then the external screw thread of the second chamber is meshed with the internal thread of the outlet 14 of the channel 12 through the rotation of the base, thereby implementing connection of the second chamber and the first chamber. When the first surface 711 of the base is in contact with the bottom of the first chamber 101, the screw threads cannot be further meshed; in this case, the external screw thread of the second chamber is meshed with the internal thread of the outlet of the channel 12, such that the base is combined with the first chamber. Of course, if the second chamber has a smooth surface rather than the external screw thread, the protruding neck-shaped pipe of the second chamber can be directly inserted near the outlet 14 of the channel 12, such that the base can be connected with the first chamber through the second chamber. In addition, in some embodiments, two areas are provided on the first surface of the base, where one area is a recess area 712 in which a cover 801 is provided; the other area is a planar area 713 in which the second chamber 708 with a protrusion is provided; the recess area is explained relative to the planar area 713 and equivalent to a sunken area in the planar area 713. When the base is combined with the bottom of the first chamber 101, the cover 801 is hidden. Of course, in order to keep the cover fixed in the recess area 712, a fixing post 714 can be provided in the recessed area, such that the cover can be sleeved on the fixing post to prevent the cover 801 from falling off during operation, and the bottom of the fixing post is located in the second surface of the base and corresponding to the protrusion 201.

Thus, when the base 102 falls off or departs from the bottom of the first chamber, the first surface of the base is exposed and reset to the state as shown FIG. 7, and the opening 710 of the second chamber is exposed. In this case, the second chamber includes the liquid sample that is taken from the first chamber and used for the primary test. The cover 801 is removed from the fixing post 714 to seal the opening 710 of the second chamber, thereby retaining the liquid sample for secondary determination. The following methods can be used to allow the base to fall off the first chamber. When the second chamber is fixed with the internal screw thread of the outlet 14 of the channel 12 through the screw thread, the base is allowed to depart from the first chamber 101 through reverse rotation of the screw thread. If the second chamber is inserted into the outlet 14 of the channel 12 in the form of a piston (for example, the outer part of the neck-shaped pipe of the second chamber on the base has a smooth surface rather than the screw thread), and an opposite force is directly applied to the base, such that the base is separated from the first chamber, that is, the neck-shaped pipe of the second chamber is separated from the outlet 14 of the channel.

As described above, the second chamber is separated from the bottom of the first chamber 101 by the base. Generally, the base is not high, about 1-2 cm. Moreover, the shape of the base fits with that of the testing chamber or the first chamber 101, and the base and the first chamber 101 are closely attached together (as shown in FIG. 1). When the base 102 is allowed to be separated from the first chamber 101, it is still inconvenient to hold the base directly by hand. As the base is not high, it is difficult to hold the base rather than the first chamber by hand; in addition, the outer surface of the base may be smooth, so it is inconvenient to apply enough force to the base. Further, when the second chamber is fixed by an object equivalent to the piston, it is even more difficult to directly pull the base out of the first chamber. The force is a longitudinally outward force, and therefore relatively large force needs to be applied to the base to implement separation.

Therefore, in order to avoid a difficulty in directly applying force to the base, in some embodiments, the present invention provides a traction mechanism, where the traction mechanism directly acts on the base, such that the base can depart from the first chamber 101 with the ease, driving the second chamber on the base to depart from the chamber cavity 101 and implementing the reservation of subsequent secondary determination liquid samples. In some embodiments, the traction mechanism is provided on the base in advance; when necessary, a pulling force is directly applied to the traction mechanism, facilitating application of an external force to the base. In some embodiments, the traction mechanism includes one pulling ring. As shown in FIG. 1, the pulling ring 301 is directly connected to the base. When the base needs to depart from the first chamber 101, the pulling ring 301 is directly pulled to allow the base to depart from the first chamber. In some embodiments, the pulling ring is connected with the base through a shaft 303; the shaft 303 is rotatably connected with the pulling ring, that is, the pulling ring 301 can rotate relative to the shaft; therefore, when the pulling ring is pulled by hand, it is convenient to rotate the pulling ring at will. The pulling ring can be passed through by fingers or other auxiliary tools, such as pencils and wooden sticks. The pulling ring 301 is pulled by the fingers or auxiliary tools, without directly acting on the base, thereby facilitating separation of the base 102 from the first chamber 101. In some embodiments, the pulling ring is fixed onto the base in advance. For example, as shown in FIG. 1 - FIG. 2, the second surface of the base, namely, the back surface thereof opposite to the first surface 711 thereof, is provided with one recess area (which is the first surface 711 formed by a relatively protruding area), and a snap-fit hole 100 is provided in the recess area, and the pulling ring is clamped in the snap-fit hole. The pulling ring is also located in the recess area, without protruding from the second surface of the base; the recess area is surrounded by surfaces 1027, 1028, 1026, 1029 of the base; the edges of the surface enclose a face, and the face is in a plane but higher than the snap-fit hole 100; therefore, when the base is engaged with the first chamber, the whole face can allow the first chamber 101 to erect on the table through the edges of the surfaces. In some ways, the pulling ring is located near the second chamber, or directly connected with the second chamber (for example, the bottom of the second chamber). As can be seen from FIG. 2 and FIG. 4, the pulling ring is directly connected with the bottom of the second chamber. This is a more preferred solution. The present invention limits neither the connection mode of the pulling ring and the base nor the direct connection position of the pulling ring and the base. In other words, the pulling ring can be directly connected with other parts of the base rather than the second chamber. However, the direct connection of the pulling ring and the second chamber is advantageous and is a most preferred mode. This is due to a fact that the base 102 is actually connected with the first chamber through the second chamber. If it is desired that the base departs from the first chamber 101, it is only necessary to allow the second chamber from the first chamber. As described previously, the neck-shaped pipe of the second chamber is inserted into the channel near the outlet 14 of the channel 12, and the base 102 can depart from the first chamber 101, provided that the neck-shaped pipe of the second chamber is separated from the channel 12. It can be understood that the neck-shaped pipe of the second chamber protruding from the first surface 711 of the base has the function to collect the liquid sample and the other function (just like a rivet) to fix the base 102 at the bottom of the first chamber to avoid falling off. Therefore, the pulling ring being directly connected with the second chamber facilitates separation of the second chamber from the first chamber, ensuring that the second chamber is not damaged. Otherwise, if the pulling ring directly acts on other parts of the base rather than the second chamber and when the pulling ring is pulled, the second chamber is not separated from the channel before the base may be allowed to depart from the bottom of the first chamber. Therefore, the neck-shaped pipe of the second chamber may be broken in the channel 12, resulting in subsequent failure to seal the second chamber. For example, the pulling ring is directly connected to the back portion of the base near the protruding post 803 of the cover. When the force is applied to the pulling ring, the part of the base near the protruding post departs from the bottom of the first chamber, but the second chamber may still be located in the outlet 14 of the channel, and the departure part of the base forms an opening angle with the outlet of the channel. If the force is continuously applied, the angle will increase. However, when receiving angular stress in the channel, the neck-shaped pipe of the second chamber may be broken and left in the channel. In this case, the liquid retained in the neck-shaped pipe of the second chamber will flow out, resulting in the leakage of the liquid sample; in addition, the opening of the second chamber cannot be sealed with the cover, thereby resulting in failure to reserve the liquid sample for secondary determination test. Therefore, in some embodiments, a connecting rod 302 is provided at the bottom of the second chamber and directly connected with the bottom of the second chamber; and the pulling ring is hinged with the connecting rod through the shaft 303, such that the pulling ring can be horizontally fixed into the snap-fit hole 100 in the recess area of the second surface of the base 102. When the pulling ring needs to be used, the pulling ring is allowed to directly depart from the snap-fit hole 100, and the shaft 303 and the connecting rod 302 are kept in a straight line, or a pulling force is directly applied from the central axis of the first chamber in a direction extending outward, such that the second chamber can directly depart from the channel 11, and the base 102 can also depart from the bottom of the first chamber.

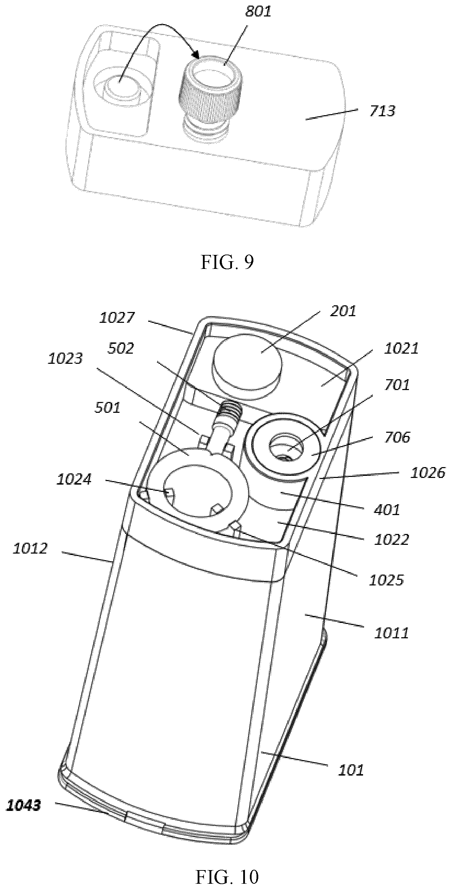

In some embodiments, one end of the connecting rod connected with the pulling ring is provided with the screw thread, and the connecting rod 302 is connected with the bottom of the second chamber through the screw thread (as shown in FIG. 2). In some embodiments, for example, as shown in FIG. 2, the second chamber 701 is an independent structure with a hollow chamber connected with the connecting rod 302, where the hollow chamber is provided with a screw thread 707, and one end of the connecting rod 302 is also provided with a screw thread, such that the second chamber is connected with the connecting rod 302 through meshing of the screw threads. The second chamber is provided with a screw thread 702 and an opening 705, and the threaded inside of the second chamber has a hollow pipe that can accommodate the liquid sample. Thus, when the opening and the screw thread are located in the channel near the outlet 14 of the channel 12, the liquid sample can flow into the hollow pipe 900 through the opening 705 to be retained. A hole 200 is provided in the base 102 and penetrates through the whole surface of the base (the plane 711 of the first surface). The bottom of the first chamber is provided with a ring 706; the ring is used to limit the position of the second chamber on the base and fix the second chamber onto the base; and the second chamber penetrates through the hole 200 and partially protrudes from the first surface, for example, the threaded part thereof also protrudes from the first surface. For example, as shown in the cross-section diagram of FIG. 5, the hole has a rim having a large inner diameter, where the rim fits with the pulling ring 706, the first chamber is sleeved with the pulling ring 706, the inner diameter of the hole 200 is reduced at the bottom of the first chamber, and the shape of hole is just like a screw; therefore, the pulling ring 706 acts on the base, such that the second chamber penetrates through the hole 200 and is in threaded connection with the inside of the outlet 14 of the channel 12 of the first chamber, the outlet 14 of the channel 12 is located in a plane 1015 where plastic strips at the bottom are located, increasing the connection stability. The screw thread of the second chamber is meshed with the screw thread of the outlet 14 of the channel 12; the opening 705 of the second chamber is located in the channel 12 and kept in fluid communication with the channel; and the screw thread of the connecting rod 302 is meshed with the screw thread of the second chamber. In addition, in some embodiments, the second chamber is provided with a sealing ring, where the sealing ring is used to form a seal between the second chamber and the hole 200 of the base to prevent liquid leakage, mainly preventing the liquid flowing into the channel 12 from leakage. When the second chamber needs to be separated from the channel 12 of the first chamber, a pulling force can be applied to the pulling ring, such that the second chamber is separated from the outlet 14 of the channel 12, implementing the separation of the second chamber from the first chamber. In such an arrangement, due to the provision of the screw threads, the pulling ring needs to be pulled with a little large force. It is necessary to reduce meshing of the screw thread 702 of the second chamber and the internal screw thread of the outlet 14 of the channel 12, such that the second chamber can be separated from the bottom of the first chamber. To facilitate the separation of the second chamber from the first chamber during threaded fixation, the following two ways can be adopted. One way is that the shaft 303 connected with the pulling ring 301 cannot rotate relative to the pulling ring, but is fixedly connected with the pulling ring; thus, when the second chamber needs to depart from the first chamber, the shaft 303 of the pulling ring 301 and the connecting rod 302 are directly kept in a straight line, and the rotation of the pulling ring causes the second chamber to rotate, thereby allowing the second chamber to be separated from the internal screw thread of the channel of the first channel. The second chamber can rotate freely in the following cases: one case is that in absence of the base 102, only the second chamber is provided, the outer rim of the pipe at the opening 705 of the second chamber is provided with the screw thread, and the pulling ring is directly connected with the second chamber. In an assembling process, the screw thread at the opening of the second chamber is meshed with the screw thread of the outlet 14 of the channel 12 through relative rotation, such as clockwise rotation. After the second chamber is connected with the first chamber, the pulling ring is fixed onto the bottom of the first chamber; the specific fixing mode thereof may be that the bottom of the first chamber is provided with the recess area in which the snap-fit hole (similar to the snap-fit hole 100 in the base), where the pulling ring is initially clamped in the snap-fit hole. After the primary test, if the second chamber needs to depart from the first chamber, the pulling ring is allowed to depart from the snap-fit hole, the shaft 303 and the connecting rod 302 are kept in a straight line, and the pulling ring is rotated counterclockwise, thereby allowing the second chamber to be separated from or depart from the first chamber. The other case is that in presence of the base, it is practicable that the second chamber can move only when rotating in the hole 200 of the base. Therefore, when the base is needed, the pulling ring 706 is clamped at the bottom of the second chamber, and then the second chamber is rotated, such that the second chamber is connected with the first chamber, and at the same time, the base is allowed to approach the bottom of the first chamber for cooperation. When the second chamber needs to depart from the first chamber, the second chamber is rotated reversely, such that the second chamber is separated from the channel 12 of the first chamber 101, the base is allowed to depart from the bottom of the first chamber, and the base and the second chamber can depart from the first chamber synchronously. In another embodiment, for example, as shown in FIG. 10, the pulling ring is separately located on the base rather than being directly connected with the second chamber. However, the pulling ring 501 is directly provided with a screw thread 502, and has two functions and is equivalent to a screwing tool; the second chamber may be likened to a screw while the pulling ring is equivalent to the screwing tool, the second chamber is rotated through the pulling ring and connected with the channel of the first chamber through the pulling ring 706, such that the base is connected with the first chamber through the second chamber. Specifically, the threaded end 502 of the pulling ring 501 is inserted into the screw thread 707 of the second chamber, the pulling ring and the second chamber are connected through relative rotation, and then the pulling ring continues to drive the second chamber to rotate, such that the external screw thread 702 of the second chamber is meshed with the internal screw thread of the outlet 14 of the channel 12, and the pulling ring rotates reversely when such meshing is completed, and is allowed to depart from the second chamber and fixed onto the base (FIG. 10). After the primary test, the pulling ring is removed again and connected with the second chamber; the second cavity is rotated reversely to depart from the channel 12 of the first chamber, such that the base and the second chamber can depart from the first chamber synchronously. In some embodiments, the second chamber is installed on the base 102 in advance, and a part thereof protrudes from the first surface of the base, and the protruding part of the second chamber is connected with the channel of the first chamber. As shown in FIG. 7, the protrusion having the screw thread and the opening is fixed onto the first surface of the base; the hole 200 is located below the opening; and a plug structure (specially named in this embodiment) is provided. For example, as shown in FIG. 3, the plug structure includes a sealing ring 704 and a protruding pipe 900 on a plug, and the whole plug also has the opening 705, and the pipe 900 is provided inside the protrusion and a part of the liquid can be accommodated in the pipe. Here, the plug structure may be separately used as the second chamber (in the specific embodiments described above), or the second chamber may be composed of the plug and the neck-shaped pipe 789 that is provided with the screw thread 709 and located on the base. Thus, when the base rotates, the opening 710 of the base is meshed with the protruding screw thread 709 and the internal screw thread of the outlet 14 of the channel 12, and the opening 705 of the plug is located below the opening 710, and the liquid flowing from the opening 710 also flows into the opening 705 and is retained in the chamber in the protruding pipe 900. When it is desired that the second chamber departs from the first chamber, the base can be rotated such that the protruding neck-shaped pipe 789 on the base departs from the channel 12 of the first chamber along with the whole plug structure, and the cover seals the opening 710 to retain the secondary determination sample. In another embodiment, the pulling ring 501 with a threaded structure is meshed with the screw thread 707 at the bottom of the plug to pull the plug away from the base; if the liquid is accommodated in the protruding pipe 900 of the plug, the opening 705 of the plug can be directly sealed, thereby directly retaining the liquid in the plug for secondary determination.

In order to facilitate the separation of the second chamber from the channel 12, the part of the second chamber protruding from the first surface 711 of the base has a smooth surface rather than the screw thread (as shown in FIG. 3). Thus, the protruding pipe 900 of the second chamber is directly inserted into the outlet 14 of the channel 12, and also has the opening 705. In this case, the screw thread is not provided inside the outlet of the channel 12, and the pipe is inserted into the channel 12 in the form of a piston, which reduces less resistance than threaded fixation. Of course, after the base and the second chamber are synchronously separated from the first chamber, the inside of the cover 801 is provided with one plug, without the screw thread. The cover is directly reconfigured at the protruding opening 705, and the plug seals the opening 705, thus sealing the second chamber.

In some embodiments, a gas channel in gas communication with the channel 12 is further provided in the extrusion table and used for exhausting excessive gas therein when the liquid flows into the channel, such that the initial liquid can smoothly flow into the second chamber through the channel. In such an arrangement, for example, when the absorption element collects saliva, the saliva itself is sticky, and a part of the saliva slowly flows into the channel 12. It is difficult for the saliva to flow in absence of pressure. In addition, the saliva flows slowly and the gas at one end of the channel 12 is sealed, and it is more difficult for the saliva to smoothly stay and immediately flow into the second chamber of the outlet 14 of the channel. In this case, some vent holes are provided in the bottom of the channel 12 or the side wall of the opening 14. Ends of the vent holes can be in gas communication with the channel 12 while the other ends thereof can penetrate through the side wall of the first channel and are in atmospheric communication with the outside (an exhaust channel is provided at a position where there are two arrows in FIG. 5), thereby immediately exhausting the gas in the channel and allowing the liquid to smoothly flow into the second chamber. Generally, gas can flow through the vent holes, but liquid cannot flow through them. In another embodiment, one end of the gas channel is in gas communication with the channel 12, and the other end thereof is in communication with the gas in the first chamber; different from that in the extrusion surface 11, the opening at the other end is provided in another place, such that the excessive gas in the channel 12 can be exhausted, and the liquid can flow quickly into the channel 12 and smoothly into the second chamber at the bottom for retention or secondary determination test.

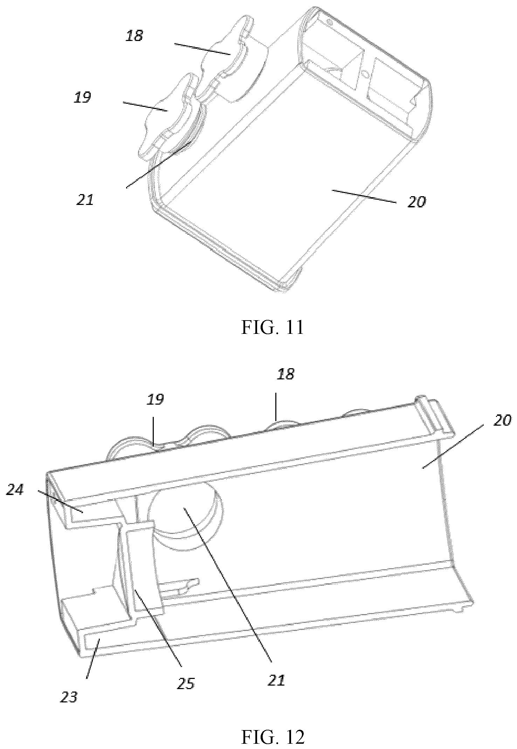

In other some embodiments, as shown in FIG. 11 and FIG. 12, one chamber 17 is provided as a primary testing chamber, and includes a testing element therein for testing an analyte in a liquid sample. Two elements are provided on a side wall 1018 of the chamber. If the shape of the chamber is similar to that in FIG. 1, the elements are provided on a narrow side wall, while test strips can be fixed onto a wide side wall for the primary test of the sample. One element is a liquid collection element 19 that is in communication with the chamber 17 through the opening 21 of the side wall; the other element 18 is the element of the cover for sealing the opening of the collection element 19; and the liquid collected by the collection element is used for the secondary determination test. During the test, the liquid sample is collected in the testing chamber 17 and obtained from the extrusion table 25. The testing device is mainly used for testing saliva. The extrusion table is used for contacting with an absorption element of a saliva collector and allowing the absorption element to release the liquid sample through compression. The liquid sample can be used for the primary test; for example, the liquid sample is present in the grooves 24, 23 and in contact with the testing element located in the grooves to complete the primary test or the liquid sample is collected in the grooves before the primary test. In this case, the opening 20 of the testing device is sealed by the cover or the like (omitted). After the opening is sealed, the testing device is erected laterally, that is, the narrow side is erected on the table, or the first chamber is inclined, such that the liquid sample in the chamber flows into the collection element 19 through the opening 21. Then, the collection element 19 is removed from the testing chamber; the cover 18 is removed from the testing chamber to seal the collection element 19; and the liquid collected by the collection element is used for the secondary determination test.

### Method for testing or collecting liquid sample

The present invention further provides a method for collecting a liquid sample, and the method includes providing the device for collecting a liquid sample, where the device includes a first chamber 101 and a second chamber; the first chamber is detachably connected with the first chamber; the first chamber 101 is used for collecting the liquid sample and performing the primary test of the liquid sample, and the liquid sample is allowed to flow into the second chamber. In some preferred embodiments, when the liquid sample is collected in the second chamber, the second chamber is separated from the first chamber, and the opening of the second chamber is sealed by the cover. In some embodiments, the second chamber is located on a base 102, and the base is connected with the first chamber through the second chamber. In some embodiments, the device includes a traction mechanism connected with the second chamber; and when the second chamber needs to depart from the first chamber, the traction mechanism is pulled to allow the second chamber to depart from the first chamber. In some embodiments, when the second chamber departs from the first chamber, the base is also separated from the first chamber.

In addition, the present invention provides a method for detecting the presence or absence of an analyte in a liquid sample; the method includes any one of the above-mentioned liquid collection devices; and after the liquid sample is collected in the first chamber, the liquid sample from the first chamber 101 is tested by the testing element. After the testing results are obtained, the second chamber is separated from the first chamber in any of the above ways. In some specific embodiments, the device further includes a testing chamber for accommodating the testing element, where the testing chamber is in fluid communication with the first chamber; and after the liquid sample is collected in the first chamber, the liquid sample flows into the testing chamber. When the testing chamber includes the testing element, the second chamber is separated from the first chamber after the testing element completes the test. In some preferred embodiments, the liquid sample flows from the first chamber into the testing chamber and then into the second chamber. Such a structure is designed as described above, such that the liquid sample flowing into the testing chamber does not flow into the second chamber and contaminate the liquid sample in the second chamber.

These embodiments are also included in this application.
1. A device for detecting an analyte in a liquid sample, comprising: a first chamber for collecting the liquid sample and a second chamber for collecting a liquid sample for determination test; wherein the first chamber is detachably combined or connected with the second chamber; the first chamber is in communication with the second chamber; and the liquid sample located in the second chamber is a part of the liquid sample in the first chamber.
2. The device according to clause 1, wherein the first chamber is in fluid communication with the second chamber; alternatively, when the second chamber does not depart from the first chamber, the first chamber is in fluid communication with the second chamber.
3. The device according to clause 2, further comprising a channel through which the first chamber is in fluid communication with the second chamber.
4. The device according to clause 3, wherein an extrusion table is provided in the first chamber; and the extrusion table is configured to allow an absorption element to be compressed on the extrusion table to release the liquid sample thereon; wherein one end of the channel is in fluid communication with the extrusion table, and the other end thereof is in fluid communication with the second chamber.
5. The device according to clause 4, further comprising a traction mechanism, wherein the traction mechanism is connected with the second chamber, and a pulling force is applied to the traction mechanism, whereby allowing the second chamber to depart from the first chamber.
6. The device according to clause 4, further comprising a base, wherein the second chamber is provided on the base, and the base is connected with the bottom of the first chamber through the second chamber.
7. The device according to clause 6, wherein one traction mechanism is provided on the base; a pulling force being applied to a traction mechanism causes the base to depart from the first chamber, whereby driving the second chamber to depart from the first chamber.
8. The device according to clause 7, wherein the traction mechanism comprises a pulling ring, a shaft for connecting the pulling ring to the base, and a connecting rod connected with the shaft, wherein the connecting rod is connected with the base.
9. The device according to clause 8, wherein the shaft and the connecting rod are foldably connected, and the pulling ring is capable to be folded and fixed onto the base.
10. The device according to clause 7, wherein the traction mechanism comprises a pulling ring; the pulling ring is provided on the base, but not connected with the base; in case of use, the pulling ring is allowed to be connected with the base such that the pulling force is applied to the pulling ring, allowing the base to depart from the first chamber.
11. The device according to clause 9, wherein a snap-fit hole is provided in the base, and the pulling ring is fixed onto the base through the snap-fit hole.
12. The device according to clause 10, wherein a snap-fit hole is provided in the base, and the pulling ring is fixed onto the base through the snap-fit hole.
13. The device according to clause 8, wherein the shaft is capable to rotate with respect to the pulling ring; alternatively, the pulling ring and the shaft are capable to be connected together through relative rotation.
14. The device according to clause 7, wherein the traction mechanism is directly connected with the base.
15. The device according to clause 14, wherein the traction mechanism is directly connected with the second chamber; the pulling force being applied to the traction mechanism causes the second chamber to depart from the first chamber, whereby driving the base to depart from the first chamber.
16. The device according to clause 15, wherein the base is provided with a first surface contacting with the bottom of the first chamber; a recess area is provided in the first surface; and a cover for sealing an opening of the second chamber is provided in the recess area.
17. The device according to clause 16, wherein a protrusion is provided in the first surface and serves as a neck-shaped pipe of the second chamber; the opening is provided in the neck-shaped pipe and used for receiving a fluid from the channel; and the neck-shaped pipe is used for connecting with an outlet of the channel.
18. The device according to clause 17, wherein the outlet of the channel is not internally provided with a screw thread; the neck-shaped pipe is also not provided with the screw thread and is directly inserted into the channel to implement connection of the second chamber and the first chamber, whereby allowing the first chamber to be in fluid communication with the second chamber.
19. The device according to clause 17, wherein the outlet of the channel is internally provided with a screw thread; the neck-shaped pipe is also provided with the screw thread; the screw thread of the neck-shaped pipe is meshed with the screw thread of the outlet of the channel to implement connection of the second chamber and the first chamber, whereby allowing the first chamber to be in fluid communication with the second chamber.
20. The device according to clause 17, wherein the screw thread of the neck-shaped pipe of the second chamber is fixed with the outlet of the channel of the first chamber through clockwise rotation; when the traction mechanism and the second chamber perform screw thread rotation, the screw thread is fixed with the second chamber through counterclockwise rotation; after fixed screw threads are used up, the traction mechanism continues to rotate counterclockwise to drive the second chamber to rotate counterclockwise, whereby allowing the second chamber to depart from the first chamber.
21. The device according to clause 19, comprising a gas communication channel thereon, wherein one end of the gas communication channel is communicated with the channel of the first chamber, while the other end thereof is communicated with outside atmosphere; and only gas is capable to pass through the gas communication channel, instead of liquid.
22. A device for detecting an analyte in a liquid sample, comprising: a first chamber for collecting the liquid sample and a second chamber for collecting a liquid sample for determination test; wherein the first chamber is detachably combined or connected with the second chamber; the first chamber is in communication with the second chamber; and the liquid sample located in the second chamber is a part of the liquid sample in the first chamber, wherein the second chamber is located on a side wall of the first chamber.
23. The device according to clause 22, wherein the side wall of the first chamber is further provided with a cover for sealing the second chamber thereon.
24. The device according to clause 22, wherein when the liquid in the first chamber needs to flow into the second chamber, the first chamber is allowed to tilt such that the liquid in the first chamber flows into the second chamber.

All the patents and publications mentioned in the description of the present invention indicate that these are public technologies in the art and can be used by the present invention. All the patents and publications cited herein are listed in the references, just as each publication is specifically referenced separately. The present invention described herein can be realized in the absence of any one element or multiple elements, one restriction or multiple restrictions, where such restriction is not specifically described here. For example, the terms "comprising", "essentially consisting of" and "consisting of" in each example herein may be replaced by the rest 2 terms. The terms and expressions which have been employed herein are descriptive rather than restrictive, and there is no intention to suggest that these terms and expressions in this description exclude any equivalents, but it is to be understood that any appropriate changes or modifications can be made within the scope of the present invention and appended claims. It can be understood that the embodiments described in the present invention are some preferred embodiments and features. Any person of ordinary skill in the art can make some modifications and changes according to the spirit of the description of the present invention. These modifications and changes are also considered to fall within the scope of the present invention and the scope limited by independent claims and dependent claims.

## Claims

1. A device for detecting an analyte in a liquid sample, comprising: a first chamber for collecting the liquid sample and a second chamber for collecting a liquid sample for determination test; wherein the first chamber is detachably combined or connected with the second chamber; the first chamber is in communication with the second chamber; and the liquid sample located in the second chamber is a part of the liquid sample in the first chamber.

2. The device according to claim 1, wherein the first chamber is in fluid communication with the second chamber; alternatively, when the second chamber does not depart from the first chamber, the first chamber is in fluid communication with the second chamber.

3. The device according to claim 2, further comprising a channel through which the first chamber is in fluid communication with the second chamber.

4. The device according to claim 3, wherein an extrusion table is provided in the first chamber; and the extrusion table is configured to allow an absorption element to be compressed on the extrusion table to release the liquid sample thereon; wherein one end of the channel is in fluid communication with the extrusion table, and the other end thereof is in fluid communication with the second chamber.

5. The device according to claim 4, further comprising a traction mechanism, wherein the traction mechanism is connected with the second chamber, and a pulling force is applied to the traction mechanism, whereby allowing the second chamber to depart from the first chamber.

6. The device according to claim 4, further comprising a base, wherein the second chamber is provided on the base, and the base is connected with the bottom of the first chamber through the second chamber.

7. The device according to claim 6, wherein one traction mechanism is provided on the base; a pulling force being applied to a traction mechanism causes the base to depart from the first chamber, whereby driving the second chamber to depart from the first chamber.

8. The device according to claim 7, wherein the traction mechanism comprises a pulling ring, a shaft for connecting the pulling ring to the base, and a connecting rod connected with the shaft, wherein the connecting rod is connected with the base.

9. The device according to claim 8, wherein the shaft and the connecting rod are foldable connected, and the pulling ring is capable to be folded and fixed onto the base.

10. The device according to claim 7, wherein the traction mechanism comprises a pulling ring; the pulling ring is provided on the base, but not connected with the base; in case of use, the pulling ring is allowed to be connected with the base such that the pulling force is applied to the pulling ring, allowing the base to depart from the first chamber.

11. The device according to claim 9, wherein a snap-fit hole is provided in the base, and the pulling ring is fixed onto the base through the snap-fit hole.

12. The device according to claim 10, wherein a snap-fit hole is provided in the base, and the pulling ring is fixed onto the base through the snap-fit hole.

13. The device according to claim 8, wherein the shaft is capable to rotate with respect to the pulling ring; alternatively, the pulling ring and the shaft are capable to be connected together through relative rotation.

14. The device according to claim 7, wherein the traction mechanism is directly connected with the base.

15. The device according to claim 14, wherein the traction mechanism is directly connected with the second chamber; the pulling force being applied to the traction mechanism causes the second chamber to depart from the first chamber, whereby driving the base to depart from the first chamber.
